# EUROPEAN PATENT APPLICATION

(11) **EP 2 987 868 A1**
(43) Date of publication of application: **24.02.2016**
(21) Application number: 14181411.1
(22) Date of filing: 19.08.2014
(51) Int. Cl.: C12Q 1/68

(54) **Electrochemical detection of single nucleotide polymorphisms**

(71) Applicant: GENSORIC GMBH, 18057 Rostock (DE)
(72) Inventor: Biala, Katarzyna, 18059 Rostock (DE); Flechsig, Gerd-Uwe, East Greenbush, NY 12061 (US); Mix, Maren, 18057 Rostock (DE); Bär, Katharina, 18258 Schwaan (DE); Krüger, Lars, 18055 Rostock (DE)
(74) Representative: Moré, Solveig Helga

(57) **Abstract**

The present invention relates to the field of DNA diagnosis, in particular, to detection of single nucleotide polymorphisms (SNPs). Single nucleotide polymorphisms play an important role in many diseases having a genetic component, e.g., in multiple sclerosis. The invention provides an electrochemical method of detection of SNP, which exploits the different temperature optima of detection of DNA of varying sequences labeled with a redox marker such as osmium tetroxide bipyridine, wherein the detection is preferably carried out at a directly heated electrode. The invention also relates to kits for carrying out said method, and a method of diagnosing a disease associated with a SNP.

SEQUENCE LISTING

<110> Gensoric GmbH

<120> Electrochemical detection of single nucleotide polymorphisms

<130> GS015539EP

<160> 16

<170> BiSSAP 1.3

<210> 1
<211> 35
<212> DNA
<213> artificial sequence

<220>
<223> capture probe

<220>
<221> modified_base
<222> 35
<223> /mod_base="OTHER"
/note="[Dithio]3"

<400> 1
caggtgtcca tacaggaagt tttttttttt ttttt 35

<210> 2
<211> 20
<212> DNA
<213> artificial sequence

<220>
<223> protective strand

<400> 2
gagcagtcca tacaggaaca 20

<210> 3
<211> 25
<212> DNA
<213> artificial sequence

<220>
<223> DNA-Target

<400> 3
acttcctgta tggacacctg ttttt 25

<210> 4
<211> 25
<212> DNA
<213> artificial sequence

<220>
<223> DNA-Target

<400> 4

acttcctgta cggacacctg ttttt                                          25


<210> 5
<211> 35
<212> DNA
<213> artificial sequence

<220>
<223> capture probe

<220>
<221> modified_base
<222> 35
<223> /mod_base="OTHER"
      /note="[Dithio]3"

<400> 5
tgctgaccta tatttcccat tttttttttt ttttt                                35


<210> 6
<211> 20
<212> DNA
<213> artificial sequence

<220>
<223> protective strand

<400> 6
aggagaccta tatttccgaa                                                 20


<210> 7
<211> 25
<212> DNA
<213> artificial sequence

<220>
<223> DNA-Target

<400> 7
atgggaaata taggtcagca ttttt                                          25


<210> 8
<211> 25
<212> DNA
<213> artificial sequence

<220>
<223> DNA-Target

<400> 8
atgggaaata caggtcagca ttttt                                          25


<210> 9
<211> 35
<212> DNA
<213> artificial sequence

<220>

<223> capture Probe

<220>
<221> modified_base
<222> 35
<223> /mod_base="OTHER"
      /note="[Dithio]3"

<400> 9
gataggaccc atattcccac tttttttttt ttttt                                35


<210> 10
<211> 20
<212> DNA
<213> artificial sequence

<220>
<223> protective strand

<400> 10
caaaggaccc atattcggag                                                 20


<210> 11
<211> 25
<212> DNA
<213> artificial sequence

<220>
<223> DNA-Target

<400> 11
gtgggaatat gggtcctatc ttttt                                           25


<210> 12
<211> 25
<212> DNA
<213> artificial sequence

<220>
<223> DNA-Target

<400> 12
gtgggaatac gggtcctatc ttttt                                           25


<210> 13
<211> 35
<212> DNA
<213> artificial sequence

<220>
<223> capture Probe

<220>
<221> modified_base
<222> 35
<223> /mod_base="OTHER"
      /note="[Dithio]3"

<400> 13

atcccagctg cgggctcacc tttttttttt ttttt 35

<210> 14
<211> 20
<212> DNA
<213> artificial sequence

<220>
<223> Protective strand

<400> 14
aaggcagctg cgggctcagg 20

<210> 15
<211> 25
<212> DNA
<213> artificial sequence

<220>
<223> DNA-Target

<400> 15
ggtgagcctg cagctgggat ttttt 25

<210> 16
<211> 25
<212> DNA
<213> artificial sequence

<220>
<223> DNA-Target

<400> 16
ggtgagcccg cagctgggat ttttt 25

## Description

The present invention relates to the field of DNA diagnosis, in particular, to detection of single nucleotide polymorphisms. Single nucleotide polymorphisms play an important role in many diseases having a genetic component, e.g., in multiple sclerosis. The invention provides an electrochemical method of detection of SNP, which exploits the different temperature optima of detection of DNA of varying sequences labeled with a redox marker such as osmium tetroxide bipyridine, wherein the detection is preferably carried out at a directly heated electrode. The invention also relates to kits for carrying out said method, and a method of diagnosing a disease associated with a SNP.

Detection of DNA by electrochemical means is taught, e.g., in WO 2007020093 A2 or US 8,216,446 (derived therefrom). It teaches a method of labelling and analysing nucleic acids, more specifically, a method of analysing nucleic acids, which comprises contacting the nucleic acids to be detected, which are in the form of single-stranded nucleic acid strands partially (i.e. over a part of the total length of the nucleic acid strands) hybridized with protective strands, with substances which specifically react with nucleobases of the single-stranded sections of the nucleic acid strands, and which are capable of subsequently participating in a reversible redox reaction in electroanalytical processes known to the skilled worker, e.g., osmium tetroxide.

A Single Nucleotide Polymorphism (SNP) is a DNA sequence variation occurring commonly within a population (e.g. 1%) in which a single nucleotide -A, C, T or G - in the genome (or other shared sequence) differs between members of a biological species or paired chromosomes. For example, two sequenced DNA fragments from different individuals contain a difference in a single nucleotide. In this case we say that there are two *alleles.* Almost all common SNPs have only two alleles. These genetic variations between individuals (particularly in non-coding parts of the genome) are exploited in DNA fingerprinting, which is used in forensic science. Also, these genetic variations underlie differences in our susceptibility to disease. The severity of illness and the way our body responds to treatments are also manifestations of genetic variations. For example, a single base mutation in the APOE (apolipoprotein E) gene is associated with a higher risk for Alzheimer disease (wikipedia).

Variations in the DNA sequences of humans can affect how humans develop diseases and respond to pathogens, chemicals, drugs, vaccines, and other agents. SNPs are also critical for personalized medicine. However, their greatest importance in biomedical research may be for comparing regions of the genome between cohorts (such as with matched cohorts with and without a disease) in genome-wide association studies. The study of SNPs is also important in crop and livestock breeding programs.

SNPs are usually biallelic and thus easily assayed. A single SNP may cause a Mendelian disease. For complex diseases, SNPs do not usually function individually, rather, they work in coordination with other SNPs to manifest a disease condition. As of 23 July 2013, dbSNP listed 62,676,337 SNPs in humans.

SNPs have been used in genome-wide association studies (GWAS), e.g. as high-resolution markers in gene mapping related to diseases or normal traits. The knowledge of SNPs will help in understanding pharmacokinetics (PK) or pharmacodynamics, i.e. how drugs act in individuals with different genetic variants. A wide range of human diseases, e.g. Sickle-cell anemia, β Thalassemia and Cystic fibrosis result from SNPs. Diseases with different SNPs may become relevant pharmacogenomic targets for drug therapy. Some SNPs are associated with the metabolism of different drugs. SNPs without an observable impact on the phenotype are still useful as genetic markers in genome-wide association studies, because of their quantity and the stable inheritance over generations (wikipedia).

Exemplary SNPs are associated, e.g., with osteoporosis (Ferron et al., 2011, Inositol Polyphosphate 4-Phosphatase B as a Regulator of Bone Mass in Mice and Humans. Cell Metabolism 14, 466-477) or with multiple sclerosis, e.g., INPP4B, which has been found to be relevant for diagnosis, prognosis and prognosis of disease progression, as well as therapy response.

Detection of SNP is usually carried out by DNA sequencing, mass spectrometry, single-strand conformation polymorphism (SSCP), restriction fragment length polymorphism or hybridization analysis. Yang et al., 2014 (Accurate Zygote-Specific Discrimination of Single-Nucleotide Polymorphisms Using Microfluidic Electrochemical DNA Melting Curves. Angew. Chem. Int. Ed. 53:3163-3167) report electrochemical detection of SNP in ApoE.

In light of this, the inventors addressed the problem of providing a novel electrochemical method of detection of SNPs. This problem is solved by the invention, in particular, by the subject matter of the claims.

The invention provides a method for detection of a Single Nucleotide Polymorphism (SNP) in a target nucleic acid, comprising
(a) hybridizing the target nucleic acid with a protective strand overlapping with the position of the (putative) polymorphism, thereby generating a partially double stranded and partially single stranded nucleic acid,
(b) labeling the partially single stranded nucleic acid by reaction with a redox marker, which reacts selectively with C-C-double bonds of the pyrimidine rings of the nucleic acid strand via Diels-Alder reaction, or wherein the redox marker is an osmium (VIII) complex, wherein the redox marker preferably is [OsO₄(bipy)],
(c) at a specific temperature, hybridizing the labeled target nucleic acid with a capture probe immobilized on the surface of an electrode, wherein the target nucleic acid has a higher affinity to the capture probe than to the protective strand, thereby displacing the protective strands,
(d) electroanalytically detecting an electrochemical signal generated by the target nucleic acid hybridizing to the capture probe.

The inventors have investigated different sequence-dependent hybridization behaviour of full and mismatched DNA targets at gold electrodes influenced by the hybridization parameters temperature, time and target concentration. The targets were modified with [OsO₄(bipy)] (osmium tetroxide bipyridine) labels using the approach of protective strands. Different DNA sequences containing SNPs that are supposed to be relevant for multiple sclerosis were investigated as examples of the method of the invention. Surprisingly, the expected hybridization behaviour that the full match target always delivers a higher signal than a single base-mismatched strand was not observed at all hybridization temperatures. These characteristics are dramatically affected by the sequence of the respective complementary (or mismatched) target and the probes.

The target nucleic acid may be RNA, but, preferably, it is DNA. The SNP in the target nucleic acid is typically biallelic. The allele of the target nucleic acid is typically unknown and is to be analyzed, but the two possible alleles are known.

Following the general principle laid out in WO 2007020093 A2 or US 8,216,446, which is fully incorporated herein by reference, the target nucleic acid, after generating single strands, e.g., by asymmetric PCR or by heating to a temperature above the melting temperature, e.g., 1-5 min at 90-95 °C, optionally followed by rapid cooling according to WO 2013/045417 A1, which is also fully incorporated herein by reference, is first hybridized with a single stranded nucleotide, the protective strand, which overlaps with the position of the SNP. Preferably, the position of the SNP is approximately central in the protective strand, i.e., the SNP's position may, independently, be located about 5, 6, 7, 8, 9, 10, 11, 12, 13, 14, or 15 nucleotides away from each end of the protective strand. The protective strand is either matched to the target strand in the position of the SNP or mismatched, which, for an unknown target, is to be determined by the method of the invention. Hybridization to the protective strand may be carried out at room temperature, e.g., preferably, 20 °C-25 °C, more preferably, about 20 °C. In the context of the invention, about means +/- 10%. Hybridization may be carried out for 30 minutes - 4 hours, 60 minutes-3 hours, or about 2 hours.

After formation of a partial double strand, i.e., a partially double stranded and partially single stranded nucleic acid, the unprotected, i.e., single stranded parts of target-strands are labeled with a redox marker as taught in WO 2007020093 A2. The redox marker only binds to thymin bases in single strands, so that the protected sequence adjacent to the SNP is not labeled, and keeps its capability of formation of a double strand. The redox marker preferably is an osmium (VIII) complex, preferably, [OsO₄(bipy)]. Alternatively, the redox marker may be a molecule consisting of an electrochemically active component and a 1,3-diene component, which reacts with C-C-double bonds of the pyrimidine rings via a Diels-Alder reaction, mimicking the osmium tetroxide bipyridine complex both sterically and electrochemically. The electrochemically active component may be selected from the group consisting of quinones, diaminobenzenes, naphtha quinones, diaminonaphthalenes, anthraquinones, phenolthia-zones, dihydroxybenzenes, phenylenediamines, tetrathiazanes, tetrathiafulvalenes and in each case the corresponding redox partners. The labeling reaction may be carried out for 30 minutes - 4 hours, 60 minutes-3 hours, or about 2 hours.

Optionally, the excess redox markers may be removed from the analytical solution by dialysis or ultrafiltration prior to the next step. Dialysis may be carried out overnight or for about 2-4 hours, wherein, optionally, the buffer is exchanged after 30 minutes -2 hours or after about 1 hour. Alternatively, excess redox markers remain in the analytical solution after labeling, and the electrochemical signals of the redox markers later bound on the surface of the electrode are separated from the signals of the redox markers present in solution during detection by suitable electrochemical analytical methods such as chronocoulometry.

After labeling, the protective strand has to be replaced by the immobilized capture probe at the electrode surface. Preferably, a gold electrode is used, but another suitable electrode, e.g., a platinum electrode, can also be employed. The capture probe may be immobilized on the electrode by a conventional method, e.g., via a dithiol-linker. Hybridization to the capture probe is carried out at a specific temperature. In the context of the invention, "a" is understood as "one or more", unless explicitly specified otherwise. Hybridization is carried out for about 10 min to about 1 hour, preferably, for about 15 min to about 30 min, most preferably, for about 15 min. A shorter time allows a higher throughput of analysis, however, the inventors showed that longer hybridization time allows for better detection, in particular, better detection of signals of matched target sequences.

To ensure higher affinity of the capture probe to the target sequence, mismatches are incorporated in each protective strand sequence, e.g., 2, 3, 4, 5, 6, 7, 8, 9 or 10 mismatches. Preferably, 4-6, most preferably, 5 mismatches are inserted in each protective strand sequence. When the SNP mismatch is located approximately in the middle of the target sequence of the protective strand complementary to the target, the mismatches in the protective strand are located near the ends of each sequence, i.e., there are at least 3, preferably, 4, 5, 6 or 7 complementary nucleotides between the location of the (potential) mismatch and the next mismatch. The length of the protective strand may be between 10 and 40 nucleotides, preferably, between 14 and 30 nucleotides, between 16 and 25 nucleotides, between 18 and 22 nucleotides or about 20 nucleotides.

The capture probe preferably does not contain any mismatches to the target nucleic acids, except, potentially, at the position of the SNP. The capture probe is further modified with a dithiol-linker at the 3'-end. The complementary sequence (including the SNP's position) may have a length of between 10 and 40 nucleotides, preferably, between 14 and 30 nucleotides, between 16 and 25 nucleotides, between 18 and 22 nucleotides or about 20 nucleotides.

Preferably, the signal is detected by voltammetric analysis.

As the inventors surprisingly found that their previous hypothesis that a matched target sequence would always lead to a higher signal than a mismatched target sequence was incorrect, comparison of the obtained signal with a standard, or with comparative target sequence is preferred. The standard can be obtained in different ways.

In one embodiment, the method of the invention comprises
(e) comparing the signal obtained for the target nucleic acid with a signal generated by carrying out steps (a)-(d) with two comparative target nucleic acids designated match-t (i.e. a target sequence complementary to the capture probe) and mismatch-t (i.e., a target sequence complementary to the capture probe except for the position of the SNP, where the mismatch-t comprises the alternative allele). Thus, match-t and mismatch-t comprise the sequence of the target nucleic acid adjacent to the SNP and preferably only differ in the polymorphous nucleotide, wherein match-t is complementary to the capture probe and mismatch-t is mismatched to the capture probe. The temperature of hybridization at step (d) is the same specific temperature for target nucleic acid, match-t and mismatch-t.

In an alternative embodiment, the method further comprises
(e) comparing the signal obtained for the target nucleic acid with a signal generated by carrying out steps (a)-(d) with a mismatched capture probe differing from the capture probe in the position of the SNP, so that the target nucleic acid is complementary either to the capture probe or to the mismatched capture probe. Thus, in this embodiment, the signal differs, as it is obtained with two different capture probes complementary to the two alleles. Optionally, the signal can be further compared with the signal generated by carrying out steps (a)-(d) with a known comparative target nucleic acid, preferably, with both capture probe and mismatch capture probe to distinguish between match and mismatch. If a sufficient number of target nucleic acids is analyzed, the different signals, in particular, the different temperature profiles, may also be distinguished without additional analysis of a known target sequence in this embodiment.

As synthesis of different capture probes is more expensive (because of the dithiol-linker modification) than synthesis of different matched or mismatched target sequences match-t or mismatch-t, the first of these two embodiments is preferred, and the invention is described according to this embodiment. However, the principles also apply to the second embodiment.

Preferably, for all targets analyzed (target nucleic acid, mismatch-t and match-t), steps (a) -(d) are performed at a plurality of temperatures. This may be performed either consecutively or in parallel. Parallel analysis is preferably carried out on an array of heated electrodes, preferably, directly heated electrodes. The capture probes are immobilized on the heated electrodes. A suitable array is, e.g., disclosed in DE 10 2011 109 402. This allows for simultaneous detection of signals at different temperatures for each target.

In one embodiment, the specific temperature is varied in the course of the detection of the signal, i.e., a temperature curve is generated on a single electrode for each target nucleic acid (and comparative nucleic acid. The temperature preferably ranges from a temperature below or at the melting temperature of the protective strand with the mismatched target sequence to a temperature above the melting temperature of the protective strand with the mismatched or matched target sequence, preferably, to at least the melting temperature of the capture probe with the mismatched target sequence. The temperature may be raised continuously, or in intervals of, e.g., 1 °C, 2 °C, 3 °C, 4 °C, 5 °C, 10 °C, 15 °C or 20 °C or any intermediate interval. The rate of heating may be, e.g., about 2 minutes per 5 °C to about 15 min per 5 °C, or about 5 min per 5°C to about 10 min per 5°C.

The method of the invention may comprise obtaining a temperature curve of signal for the target nucleic acid, and comparative temperature curves for mismatch-t and match-t or for the comparative capture probe, at a plurality of temperatures. The temperature curve of the target nucleic acid is then compared to the comparative temperature curves. If it matches the temperature curve for the mismatch, it can be concluded that the target nucleic acid comprises a mismatch, and if it matches the temperature curve of the match, it can be concluded that the target nucleic acid comprises a match.

The plurality of temperatures at which the hybridization with the capture probe is performed may comprise at least a temperature below or at the melting temperature of the protective strand to the mismatched or matched target sequence and a temperature of at least the melting temperature of the capture probe to the mismatched or matched target sequence, and, optionally, a temperature between the melting temperature of the protective strand to the mismatched target sequence and the melting temperature of the capture probe to the mismatched target sequence.

The plurality of temperatures at which the hybridization with the capture probe is performed may alternatively comprise at least a temperature below or at the melting temperature of the protective strand to the mismatched or matched target sequence and a temperature between the melting temperature of the protective strand to the mismatched target sequence and the melting temperature of the capture probe to the mismatched target sequence, and, optionally, a temperature of at least the melting temperature of the capture probe to the mismatched or matched target sequence.

The plurality of temperatures comprises two, three, four, five, six, seven, eight, nine, ten, 11, 12, 13, 14, 15, 16, 17, 18, 19, 20 or more temperatures, preferably, about 4-8 or 5-7 or 6 temperatures, e.g., with intervals or 1 ° C, 2° C, 3° C, 4° C, 5° C, 6° C, 7° C, 8° C, 9 °C, 10° C, 15 °C or 20 °C, preferably, intervals of about 5 °C-10 ° C. The range may be, e.g., between 30°C and 70°C, 35 and 65°C, 40 and 60°C, 45 and 55°C or 50 and 55°C. As described below, specific temperatures and ranges are preferred for specific SNPs. The melting temperatures of protective strands and capture probes may serve as guidance in the selection of appropriate temperatures.

Melting temperature of a double strand consisting of two nucleic acid strands acid may be determined as known in the state of the art. For example, they may be calculated by summing up 2°C for each complementary T or A and 4°C for each complementary G or C in the sequence of one of the two nucleic acids. Melting curve analysis is a possible experimental method to find the melting temperature due to Surkus and Flechsig, Electrochemical detection of DNA melting curves by means of heated biosensors, Electroanalysis 21 (2009) 1119; or Yang et al., 2014 (Accurate Zygote-Specific Discrimination of Single-Nucleotide Polymorphisms Using Microfluidic Electrochemical DNA Melting Curves. Angew. Chem. Int. Ed. 53:3163-3167). It is essential to perform the melting curve analysis at an electrode surface, because in bulk solution the Tm values can be 30° C higher.

The melting temperatures for the protective strand and the target nucleic acid are usually in the range of about 38-50°C, preferably, about 40-44°C (with higher melting temperatures for the matched than for the mismatched allele of the SNP). The melting temperatures for the capture probe to the target nucleic acid are typically about 52-68°C, preferably about 54-60°C (again, with higher melting temperatures for the matched than for the mismatched allele of the SNP).

The inventors found that, surprisingly, the signal of a mismatched target sequence may be higher than the signal of a matched target sequence. This may be the case for hybridization at or below the melting temperature of the protective strand to the mismatched target sequence. For example, this applies if the SNP is selected from the group comprising PTPN22, CD58, and HSChr16.

At other temperatures of hybridization, the signal of a matched target sequence may be higher than the signal of a mismatched target sequence. This may be the case at the melting temperature of the capture probe to the mismatched target sequence, or higher than that temperature. For example, this applies if the SNP is selected from the group comprising HLA-DPB1, PTPN22, CD58, and HSChr16.

Therefore, in the method of the present invention, after determination of one or two optimal temperatures for differentiation between matched and mismatched target sequence, the hybridization may also be carried out at said one or two specific temperature(s) without analyzing a full temperature range. For example, for detection of HLA-DPB1, the temperature is about 50°C, for detection of PTPN22, the temperature is about 30°C and/or about 55°C, for detection of CD58, the specific temperature is about 40°C and/or about 50-55°C, and, for detection of HSChr16, the temperature is about 50-55°C and/or about 65°C. If two temperatures are provided for each SNP, the lower temperature of these leads to a higher signal for a mismatch, and the higher temperature leads to a higher signal for a match.

While the previous steps (a)-(c) may be carried out at room temperature, the temperature of the hybridization to the capture probe in step (d) is a defined temperature, which, usually, is higher than room temperature. This temperature may be achieved by heating the complete reaction mix. It may also be achieved by heating the electrode at which the capture probe is immobilized. Preferably, the electrode is a directly heated electrode. In an array, each directly heated electrode may be heated individually. Alternatively, the array may be heated in total, e.g., for generating a temperature curve with the one or more target nucleic acids and the comparative nucleic acids (mismatch-t and match-t).

In a preferred embodiment, the method of the invention is carried out after amplification of the target nucleic acid or a region thereof comprising the SNP. Preferably, the nucleic acid is amplified by PCR. The PCR may be symmetric or asymmetric, and may yield a high number of single stranded target nucleic acid sequences.

The concentration of the target nucleic acid may be known. For example, the concentration may be standardized after PCR. The concentration can, e.g., be detected by optical means, or it can also be determined electrochemically, e.g., after dilution, as the inventors have shown that the signal is concentration dependent in certain ranges. A standard curve for determination of the concentration may be employed. Electrochemical detection of the concentration may be carried out with the same protective strand/capture probe as detection of the SNP, or can be carried out based on a different sequence present in the PCR sequence, which has the advantage of being independent on the SNP comprised in the analyzed target nucleic acid. If so, it is advantageous to determine signals for a concentration of comparative respective nucleic acids of known concentration in parallel.

Of note, as the temperature-dependency of the signal detected in the method of the invention (e.g. The optimum of detection for match or mismatch) is not dependent on the concentration, the presence or absence of the mismatch in the target nucleic acid may also be determined if the concentration of the target nucleic acid is unknown.

Preferably, in particular if the concentration of target nucleic acid is unknown, the method of the invention is carried out at a plurality of temperatures, e.g., 5-7 temperatures with an interval of about 5 °C, as shown, e.g., in the figures. The signal can then be normalized, e.g., with regard to the lowest or highest signal detected; preferably, the highest signal. Accordingly, the form of the signal-temperature curves is characteristic for match or mismatch in the target nucleic acid. In a preferred embodiment, the signals at the selected temperatures are simultaneously determined on an array of individually heated electrodes, preferably, directly heated electrodes, as disclosed above, which makes detection of SNPs much quicker than the prior art methods, e.g., in Yang et al. 2014.

The invention also relates to a method of diagnosing a disease associated with a SNP in a patient, or a method of assessing the risk of a patient for developing a disease, or for determining the risk of progression of a disease, wherein the disease is associated with one or more SNP, the method comprising detecting presence or absence of the one or more SNP in a patient by the method of the invention.

If the disease is multiple sclerosis, the SNPs may be selected from the group comprising HLA-DPB1, PTPN22, CD58, INPP4b and HSChr16, preferably; HLA-DPB1, PTPN22, CD58, INPP4b and HSChr16.

In case the patient is heterozygous for a SNP, the signal detected will be an overlay of the two signals of the respective alleles. In the case of different temperature optima of the matched and mismatched alleles in the method of the invention, a distinct, two-peaked signal will be obtained in a temperature curve. Data analysis may allow for differentiation of heterozygous from homozygous patients.

The present invention also provides a kit comprising:
- a protective strand, a redox marker, a capture probe and mismatch-t and match-t nucleotides suitable for detection of a SNP by the method of any of the preceding claims, and, optionally, an electrode, which is preferably incorporated in an array of electrodes, wherein the capture probe is optionally immobilized on the electrode(s), or
- a protective strand, a redox marker, a match-capture probe and a mismatch-capture probe and a comparative target nucleic acid suitable for detection of a SNP by the method of any of the preceding claims, and, optionally, an electrode, which is preferably incorporated in an array of electrodes, wherein the capture probe is optionally immobilized on the electrode(s).

The electrodes may be heatable or heated, preferably, directly heatable or directly heated. The capture probe may be immobilized on the electrode or electrodes, or it may be suitable for immobilization. The redox marker preferably is [OsO₄(bipy)]. The protective strand and capture probe may be one or more, e.g., all of the capture probes provided in the examples below. The respective target nucleic acids (with match and mismatch) provided in the examples below can also be incorporated in the kit and used as comparative targets.

The invention also provides use of a kit of the invention for detecting a SNP, or for diagnosing a disease, assessing the risk of a patient for developing a disease (prognosis of a disease), or for determining the risk of progression of a disease, or for determining the probable response to a therapy, wherein the disease is associated with one or more SNP.

All references cited herein are fully incorporated herein. The examples are merely intended to illustrate the invention, not to limit its scope.

### Figure legends

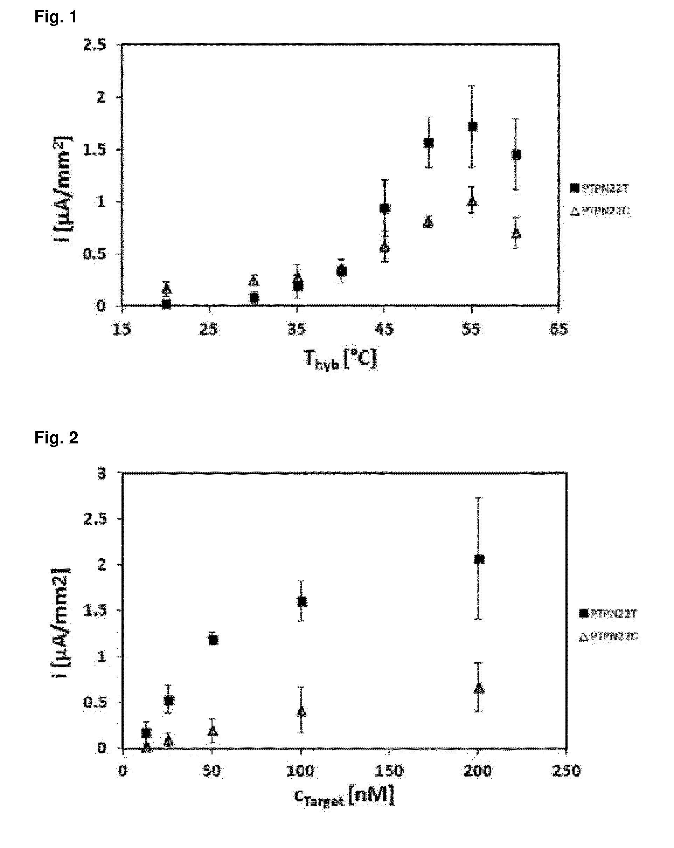

**Fig. 1****.** Influence of hybridization temperature on square-wave voltammetric signals at gold disc electrodes in Tris-buffer after 15 min of hybridization in 0.2 µM fully complementary target PTPN22T (squares) and 0.2 µM target PTPN22C with one mismatch (triangles). T_{Hyb} represents the temperature of the target solution during the hybridization step. SWV conditions: frequency 200 Hz; amplitude 40 mV. During the hybridization step the solution was stirred by a magnetic stirrer. The error bars indicate standard deviations of three independent repetitive measurement series, each obtained with a newly prepared probe SAM. Signals were Savitzky and Golay smoothed and baseline-corrected.
**Fig. 2****.** Influence of target concentration on square-wave voltammetric signals at gold disc electrodes in Tris-buffer after 15 min of hybridization in fully complementary target PTPN22T (squares) and target PTPN22C with one mismatch (triangles) at 55 °C. c_{Target} represents the concentration of the target solution during the hybridization step. SWV conditions: frequency 200 Hz; amplitude 40 mV. During the hybridization step the solution was stirred by a magnetic stirrer. The error bars indicate standard deviations of three independent repetitive measurement series, each obtained with a newly prepared probe SAM. Signals were Savitzky and Golay smoothed and baseline-corrected.

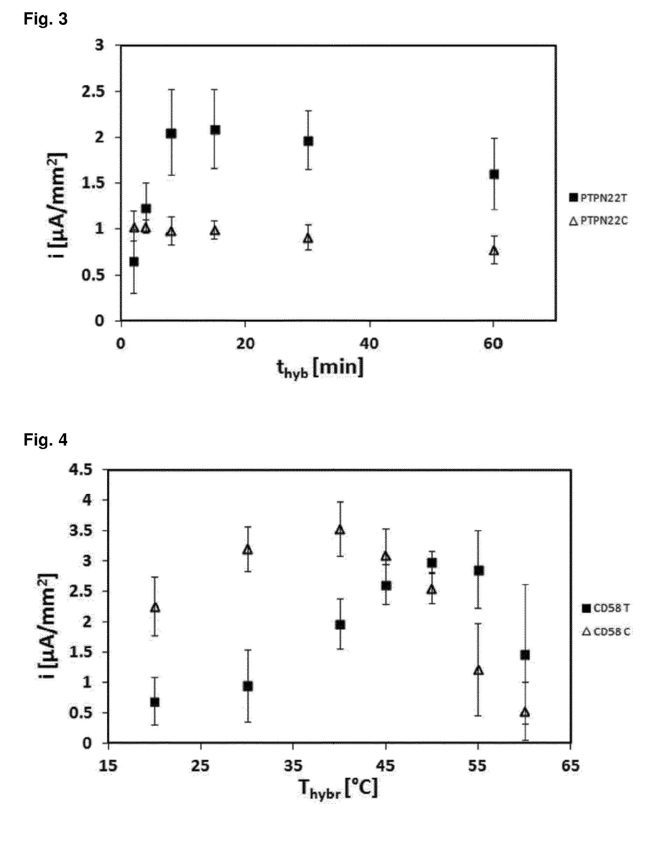

**Fig. 3****.** Influence of hybridization time (t_{hyb}) on square-wave voltammetric signals at gold disc electrodes in Tris-buffer after hybridization in 0.2 µM fully complementary target PTPN22T (squares) and 0.2 µM target PTPN22C with one mismatch (triangles) at 55 °C. SWV conditions: frequency 200 Hz; amplitude 40 mV. During the hybridization step the solution was stirred by a magnetic stirrer. The error bars indicate standard deviations of three independent repetitive measurement series, each obtained with a newly prepared probe SAM. Signals were Savitzky and Golay smoothed and baseline-corrected.
**Fig. 4** Influence of hybridization temperature on square-wave voltammetric signals at gold disc electrodes in Tris-buffer after 15 min of hybridization in 0.2 µM fully complementary target CD58T (squares) and 0.2 µM target CD58C with one mismatch (triangles). T_{Hyb} represents the temperature of the target solution during the hybridization step. SWV conditions: frequency 200 Hz; amplitude 40 mV. During the hybridization step the solution was stirred by a magnetic stirrer. The error bars indicate standard deviations of three independent repetitive measurement series, each obtained with a newly prepared probe SAM. Signals were Savitzky and Golay smoothed and baseline-corrected.

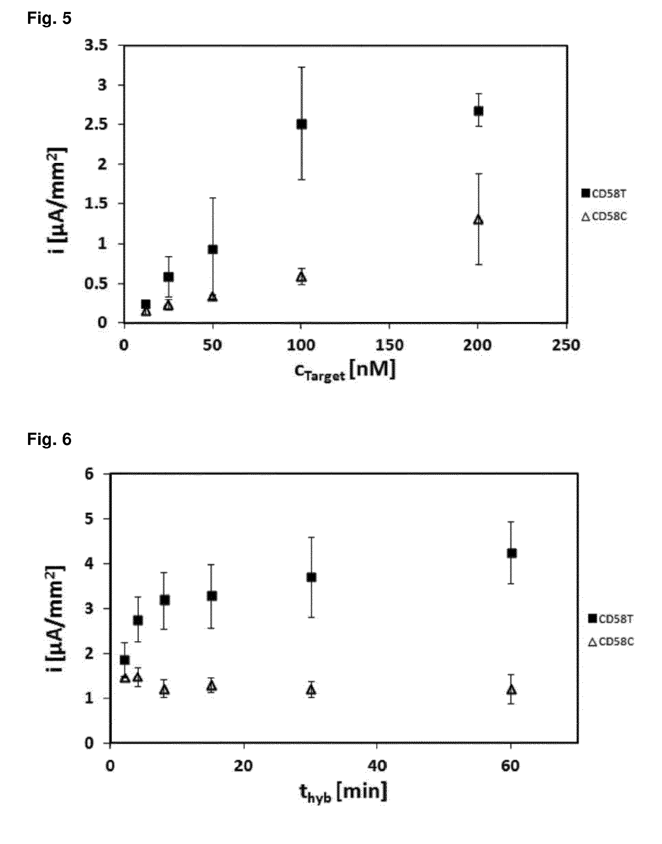

**Fig. 5** Influence of target concentration on square-wave voltammetric signals at gold disc electrodes in Tris-buffer after 15 min of hybridization in fully complementary target CD58T (squares) and target CD58C with one mismatch (triangles) at 55 °C. c_{Target} represents the concentration of the target solution during the hybridization step. SWV conditions: frequency 200 Hz; amplitude 40 mV. During the hybridization step the solution was stirred by a magnetic stirrer. The error bars indicate standard deviations of three independent repetitive measurement series, each obtained with a newly prepared probe SAM. Signals were Savitzky and Golay smoothed and baseline-corrected.
**Fig. 6** Influence of hybridization time (t_{hyb}) on square-wave voltammetric signals at gold disc electrodes in Tris-buffer after hybridization in 0.2 µM fully complementary target CD58T (squares) and 0.2 µM target CD58C with one mismatch (triangles) at 55 °C. SWV conditions: frequency 200 Hz; amplitude 40 mV. During the hybridization step the solution was stirred by a magnetic stirrer. The error bars indicate standard deviations of three independent repetitive measurement series, each obtained with a newly prepared probe SAM. Signals were Savitzky and Golay smoothed and baseline-corrected.

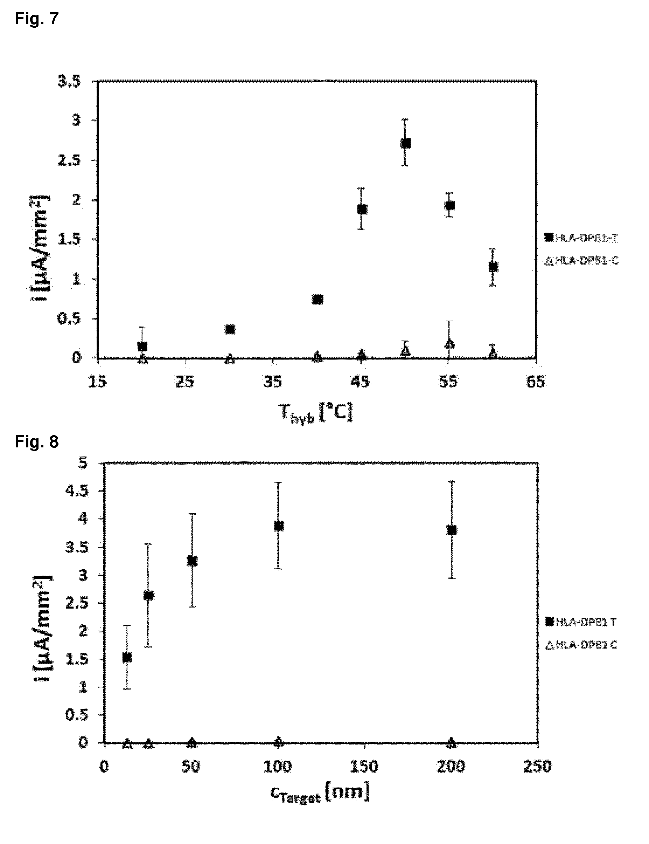

**Fig. 7** Influence of hybridization temperature on square-wave voltammetric signals at gold disc electrodes in Tris-buffer after 15 min of hybridization in 0.2 µM fully complementary target HLA-DPB1-T (squares) and 0.2 µM target HLA-DPB1-C with one mismatch (triangles). T_{Hyb} represents the temperature of the target solution during the hybridization step. SWV conditions: frequency 200 Hz; amplitude 40 mV. During the hybridization step the solution was stirred by a magnetic stirrer. The error bars indicate standard deviations of three independent repetitive measurement series, each obtained with a newly prepared probe SAM. Signals were Savitzky and Golay smoothed and baseline-corrected.
**Fig. 8** Influence of target concentration on square-wave voltammetric signals at gold disc electrodes in Tris-buffer after 15 min of hybridization in fully complementary target HLA-DPB1-T (squares) and target HLA-DPB1-C with one mismatch (triangles) at 50 °C. c_{Target} represents the concentration of the target solution during the hybridization step. SWV conditions: frequency 200 Hz; amplitude 40 mV. During the hybridization step the solution was stirred by a magnetic stirrer. The error bars indicate standard deviations of three independent repetitive measurement series, each obtained with a newly prepared probe SAM. Signals were Savitzky and Golay smoothed and baseline-corrected.

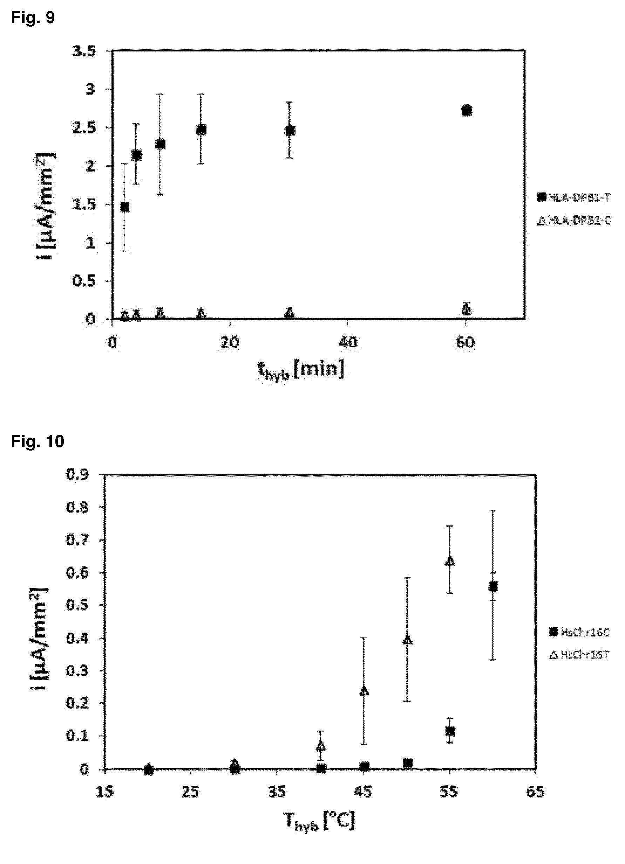

**Fig 9** Influence of hybridization time (t_{hyb}) on square-wave voltammetric signals at gold disc electrodes in Tris-buffer after hybridization in 0.2 µM fully complementary target HLA-DPB1-T (squares) and 0.2 µM target HLA-DPB1-C with one mismatch (triangles) at 50 °C. SWV conditions: frequency 200 Hz; amplitude 40 mV. During the hybridization step the solution was stirred by a magnetic stirrer. The error bars indicate standard deviations of three independent repetitive measurement series, each obtained with a newly prepared probe SAM. Signals were Savitzky and Golay smoothed and baseline-corrected.
**Fig. 10** Influence of hybridization temperature on square-wave voltammetric signals at gold disc electrodes in Tris-buffer after 15 min of hybridization in 0.2 µM fully complementary target HsChr16C (squares) and 0.2 µM target HsChr16T with one mismatch (triangles). T_{Hyb} represents the temperature of the target solution during the hybridization step. SWV conditions: frequency 200 Hz; amplitude 40 mV. During the hybridization step the solution was stirred by a magnetic stirrer. The error bars indicate standard deviations of three independent repetitive measurement series, each obtained with a newly prepared probe SAM. Signals were Savitzky and Golay smoothed and baseline-corrected.

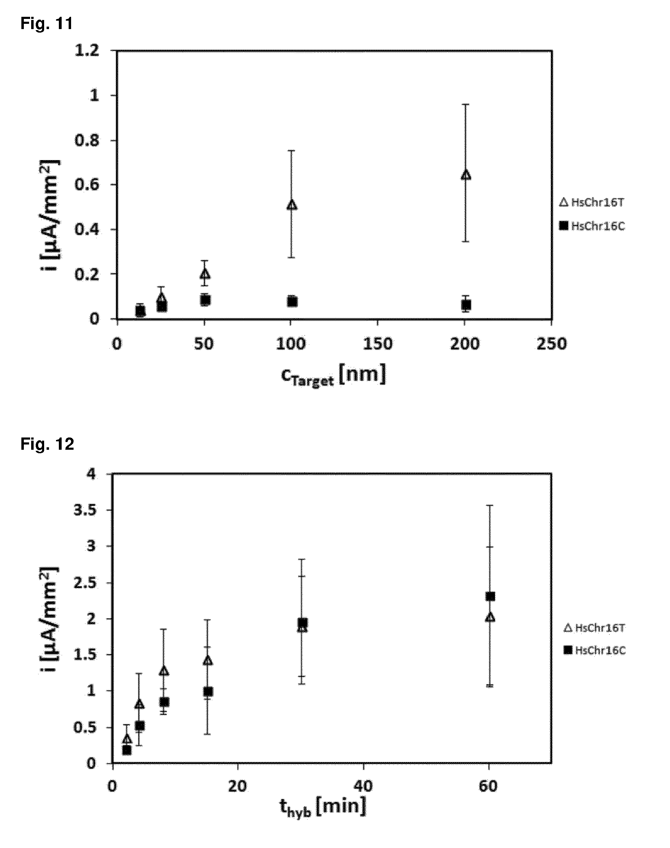

**Fig. 11** Influence of target concentration on square-wave voltammetric signals at gold disc electrodes in Tris-buffer after 15 min of hybridization in fully complementary target HsChr16C (squares) and target HsChr16T with one mismatch (triangles) at 55 °C. c_{Target} represents the concentration of the target solution during the hybridization step. SWV conditions: frequency 200 Hz; amplitude 40 mV. During the hybridization step the solution was stirred by a magnetic stirrer. The error bars indicate standard deviations of three independent repetitive measurement series, each obtained with a newly prepared probe SAM. Signals were Savitzky and Golay smoothed and baseline-corrected.
**Fig. 12** Influence of hybridization time (t_{hyb}) on square-wave voltammetric signals at gold disc electrodes in Tris-buffer after hybridization in 0.2 µM fully complementary target HsChr16C (squares) and 0.2 µM target HsChr16T with one mismatch (triangles) at 55 °C. SWV conditions: frequency 200 Hz; amplitude 40 mV. During the hybridization step the solution was stirred by a magnetic stirrer. The error bars indicate standard deviations of three independent repetitive measurement series, each obtained with a newly prepared probe SAM. Signals were Savitzky and Golay smoothed and baseline-corrected.

### Examples

### Material

CP: capture probe
PS: Protective strand

### PTPN22

CP PTPN22: 5'-CAGGTGTCC**A**TACAGGAAGTTTTTTTTTTTTTTTT[Dithio]₃-3' (SEQ ID NO: 1)
Protective strand PTPN22: 5'-**G**AG**CA**GTCCATACAGGAA**CA**-3' (SEQ ID NO: 2)
DNA-Target PTPN22T: 5'-ACTTCCTGTATGGACACCTGTTTTT-3' (SEQ ID NO: 3)
DNA-Target PTPN22C: 5'-ACTTCCTGTACGGACACCTGTTTTT-3' (SEQ ID NO: 4)

### Melting temperature:

Target T-CP: 8*4+12*2=56°C
Target C-CP: 8*4+10*2=54°C
Target T-PS: 6*4+9*2=42°C
Target C-PS: 6*4+ 8*2=40°C

### CD58

CP CD58: 5'-TGCTGACCT**A**TATTTCCCATTTTTTTTTTTTTTTT[Dithio]₃-3' (SEQ ID NO: 5)
Protective strand CD58: 5'-**A**G**GA**GACCTATATTTCC**G**A**A**-3' (SEQ ID NO: 6)
DNA-Target CD58T: 5'-ATGGGAAATATAGGTCAGCATTTTT-3' (SEQ ID NO: 7)
DNA-Target CD58C: 5'-ATGGGAAATACAGGTCAGCATTTTT-3' (SEQ ID NO: 8)

### Melting temperature:

Target T-CP: 8*4+12*2=56°C
Target C-CP: 8*4+10*2=54°C
Target T-PS: 6*4+9*2=42°C
Target C-PS: 6*4+ 8*2=40°C

### HLA-DPB1

Capture Probe HLA-DPB1: 5'-GATAGGACCC**A**TATTCCCACTTTTTTTTTTTTTTT[Dithio]₃-3' (SEQ ID NO: 9)
Protective strand HLA-DPB1: 5'-**C**A**A**AGGACCCATATTC**GG**A**G**-3 (SEQ ID NO: 10)
DNA-Target HLA-DPB1T: 5'-GTGGGAATATGGGTCCTATCTTTTT-3' (SEQ ID NO: 11)
DNA-Target HLA-DPB1C: 5'-GTGGGAATACGGGTCCTATCTTTTT-3' (SEQ ID NO: 12)

### Melting temperature:

Target T-CP: 10*4+10*2=60°C
Target C-CP: 10*4+ 9*2=58°C
Target T-PS: 6*4+9*2=42°C
Target C-PS: 6*4+ 8*2=40°C

### HSChr16

Capture Probe HSChr16: 5'-ATCCCAGCTGC**G**GGCTCACCTTTTTTTTTTTTTTT[Dithio]₃-3' (SEQ ID NO: 13)
Protective strand HSChr16: 5'-**A**A**GG**CAGCTGCGGGCTCA**GG**-3' (SEQ ID NO: 14)
DNA-Target HSChr16T: 5'-GGTGAGCC**T**GCAGCTGGGATTTTTT-3' (SEQ ID NO: 15)
DNA-Target HSChr16C: 5'-GGTGAGCC**C**GCAGCTGGGATTTTTT-3' (SEQ ID NO: 16)

### Melting temperature:

Target T-CP: 13*4+ 6*2=64°C
Target C-CP: 14*4+ 6*2=68°C
Target T-PS: 9*4+ 5*2=46°C
Target C-PS: 10*4+ 5*2=50°C

2,2'-Bipyridine was delivered by Merck and osmium tetroxide was obtained as a 2% aqueous solution from Fluka. The Tris-buffer containing 10mM tris-(hydroxymethyl)-aminomethane and 0.5 M Na₂SO₄ was adjusted to pH 7.5 using sulfuric acid. All DNA oligonucleotides were delivered by Friz Biochem (Neuried, Germany). The probe strand contained 15 thymine bases as a spacer to allow high hybridization efficiency on the electrode surface. All electrochemical measurements were performed in a 3-electrode system using a µ-Autolab III, Metrohm AG, Filderstadt, Germany (for measurements with PTPN22 Sequence), Autolab PGSTAT 128 (for measurements with Sequences: CD58, HLA-DPB1, HSChr16). The used reference electrode was an Ag/AgCl electrode (Metrohm) and the counter electrode was a glassy carbon electrode (Metrohm). A modified gold disc electrode (Diameter 2mm and 3mm, Metrohm) has been used as the working electrode. The bulk solution temperature during the hybridization process was controlled by means of a jacketed cell and a cryostat (Huber).

### Target and electrode preparation

The DNA-Targets were labeled with [OsO₄(bipy)] by means of protective strands. In the first step, 25 µl unlabelled DNA-Target solution and 25 µl protective strand solution (each 100 µM dissolved in Tris-buffer were mixed together and left for 2 hours at room temperature to allow hybridization. Then 12.5 µl of 10 mM [OsO₄(bipy)] were added to label the five unprotected 5'-terminal thymine bases and left again for 2 hours at room temperature. This way, the recognition site of the signaling strand remained unmodified. To remove the excess of [OsO₄(bipy)], the solution was given into a dialysis cell (Slide-A-Lyser Mini Dialysis Units, USA, Rockford). Dialysis was performed against Tris-buffer for one hour and after refreshing the buffer overnight at 4 °C. After the dialysis, the solution containing the [OsO₄(bipy)]-labeled target was used for the electrochemical experiments.

Before each measurement, the gold disc electrode was polished using corundum 0.3 µm (Buehler) and thoroughly rinsed with deionised water. After this, the electrode was cleaned electrochemically by using cyclic voltammetry (25 scans, -0.3 to 1.7 V, scan rate 100 mV/s) in 0.5 M sulfuric acid. After rinsing the electrode with water, the electrode was dried under a stream of nitrogen. In the next step, a 4 µl or 10 µl drop of the probe solution (30 µM in Tris-buffer) was placed onto the top of the cleaned electrode surface in order to form the capture probe SAM. This volume was sufficient to ensure complete coverage. After 16 hours at room temperature in a water-saturated atmosphere, the electrode was immersed in 1 mM aqueous 6-mercapto-1-hexanol solution for one hour to obtain a well ordered surface with immobilized DNA probes in an upright position. After rinsing this ssDNA-SAM-modified-electrode with water, it was used for hybridization experiments with the target strands.

### Measurements

All electrochemical measurements were performed at room temperature in the Tris-buffer using square-wave voltammetry (SWV) with the following parameters: start potential -0.55 V, end potential 0 V, amplitude 40 mV, frequency 200 Hz. For the hybridization the electrode was dipped in a target solution containing the labeled DNA target. After the voltammetric measurements, the electrode was immersed into 50 °C water for 60 s to dehybridize the labeled target. After detecting the dehybridization signal, the electrode was used again for another hybridization reaction at another temperature/ target solution concentration/hybridization time. After the last dehybridization the electrode was cleaned as described above. The peak-shaped measuring signals were Savitzky and Golay smoothed and baseline-corrected using the GPES 4.9 software.

### Results

Of the investigated SNP sequences, only HLA-DPB1 behaved as previously expected by the inventors. The SNP-related mismatch target always delivers a much smaller signal compared with the full match. This applies to all studied hybridization temperatures, concentrations and times. The 3 other investigated SNP sequences showed a somewhat paradox behavior. The most extreme case is that of sequence CD58: up to 45 °C, the mismatch, i.e. the SNP, yields much larger signals than the full match, at least it is possible to distinguish between both of them. Between 45 °C and 50 °C, no discrimination is possible. Only at 55 °C and above, the match yields a larger signal, as previously expected.

## Claims

1. A method for detection of a Single Nucleotide Polymorphism (SNP) in a target nucleic acid, comprising
(a) hybridizing the target nucleic acid with a protective strand overlapping with the position of the (putative) polymorphism, thereby generating a partially double stranded and partially single stranded nucleic acid,
(b) labeling the partially single stranded nucleic acid by reaction with a redox marker, which reacts selectively with C-C-double bonds of the pyrimidine rings of the nucleic acid strand via Diels-Alder reaction, or wherein the redox marker is an osmium (VIII) complex, wherein the redox marker preferably is [OsO₄(bipy)],
(c) at a specific temperature, hybridizing the labeled target nucleic acid with a capture probe immobilized on the surface of an electrode, wherein the target nucleic acid has a higher affinity to the capture probe than to the protective strand, thereby displacing the protective strands,
(d) electroanalytically detecting an electrochemical signal generated by the target nucleic acid hybridizing to the capture probe.

2. The method of claim 1, further comprising
(e) comparing the signal obtained for the target nucleic acid with a signal generated by carrying out steps (a)-(d) with two comparative target nucleic acids designated match-t and mismatch-t, wherein match-t and mismatch-t comprise the sequence of the target nucleic acid adjacent to the SNP and differ in the polymorphous nucleotide, wherein match-t is complementary to the capture probe and mismatch-t is mismatched to the capture probe, wherein the temperature at step (d) is the same specific temperature.

3. The method of claim 1, further comprising
(e) comparing the signal obtained for the target nucleic acid with a signal generated by carrying out steps (a)-(d) with a mismatched capture probe differing from the capture probe in the position of the SNP, so that the target nucleic acid is complementary either to the capture probe or to the mismatched capture probe, and, optionally, comparing the signal with the signal generated by carrying out steps (a)-(d) with a known comparative target nucleic acid, preferably, with both capture probe and mismatch capture probe.

4. The method of any of the preceding claims, comprising performing steps (a) -(d) at a plurality of temperatures, either consecutively or in parallel, preferably, in parallel on an array of directly heated electrodes with capture probes immobilized thereon.

5. The method of any of the preceding claims, wherein the specific temperature is varied in the course of the detection of the signal, wherein the temperature preferably ranges from a temperature below or at the melting temperature of the protective strand to the mismatched target sequence to a temperature of at least the melting temperature of the capture probe to the mismatched target sequence.

6. The method of any of the preceding claims, comprising obtaining a temperature curve of the signal for the target nucleic acid, and comparative temperature curves for mismatch-t and match-t or for the comparative capture probe, at a plurality of temperatures, and comparing the temperature curve of the target to the comparative temperature curves.

7. The method of any of claims 4 or 5, wherein the plurality of temperatures comprises at least a temperature below or at the melting temperature of the protective strand to the mismatched target sequence and a temperature of at least the melting temperature of the capture probe with the mismatched target sequence, and, optionally, a temperature between the melting temperature of the protective strand with the mismatched target sequence and the melting temperature of the capture probe with the mismatched target sequence.

8. The method of any of the preceding claims, wherein the electrode is a heated electrode, preferably, a directly heated electrode.

9. The method of any of the preceding claims, wherein the signal of a mismatched target sequence is higher than the signal of a matched target sequence at or below the melting temperature of the protective strand with the mismatched target sequence, wherein the SNP is selected from the group comprising PTPN22, CD58, and HSChr16.

10. The method of any of the preceding claims, wherein the signal of a matched target sequence is higher than the signal of a mismatched target sequence at or higher than the melting temperature of the capture probe with the mismatched target sequence, wherein the SNP is selected from the group comprising HLA-DPB1, PTPN22, CD58, and HSChr16.

11. The method of any of the preceding claims, wherein, after determination of one or two optimal temperatures for differentiation between matched and mismatched target sequence, the detection is carried out at said one or two specific temperature(s).

12. The method of claim 10, wherein, for detection of HLA-DPB1, the temperature is about 50°C, for detection of PTPN22, the temperature is about 30°C and/or about 55°C, for detection of CD58, the specific temperature is about 40°C and/or about 50-55°C, and, for detection of HSChr16, the temperature is about 50-55°C and/or about 65°C.

13. The method of any of the preceding claims, wherein the method is carried out after amplification of the target nucleic acid or a region thereof comprising the SNP, preferably, by PCR.

14. A method of diagnosing a disease, of assessing the risk of a patient for developing a disease, of determining the risk of progression of a disease, or of determining the probable response to a therapy of a disease in a patient, wherein the disease is associated with a SNP, comprising detecting presence or absence of the SNP in the patient by the method of any of the preceding claims, wherein, preferably, the disease is multiple sclerosis and the SNPs are selected from the group comprising HLA-DPB1, PTPN22, CD58, INPP4b and HSChr16.

15. A kit comprising:
- a protective strand, a redox marker, a capture probe and mismatch-t and match-t nucleotides suitable for detection of a SNP by the method of any of the preceding claims, and, optionally, a directly heated electrode, which is preferably incorporated in an array of directly heated electrodes, wherein the capture probe is optionally immobilized on the electrode(s), or
- a protective strand, a redox marker, a match-capture probe and a mismatch-capture probe and a comparative target nucleic acid suitable for detection of a SNP by the method of any of the preceding claims, and, optionally, a directly heated electrode, which is preferably incorporated in an array of directly heated electrodes, wherein the capture probe is optionally immobilized on the electrode(s).
